# EUROPEAN PATENT APPLICATION

(11) **EP 4 822 035 A1**
(43) Date of publication of application: **14.10.2026**
(21) Application number: 25747471.8
(22) Date of filing: 09.01.2025
(51) Int. Cl.: C12Q 1/6886, C12Q 1/686, C12N 15/11

(54) **METHYLATED GENE MARKER COMBINATION FOR EARLY DETECTION OF ENDOMETRIAL CANCER AND USE THEREOF, AND MULTIPLEX PCR DETECTION KIT AND USE THEREOF**

(30) Priority: 02.02.2024 CN 202410149219
(71) Applicant: Sun Yat-Sen University Cancer Center, Guangzhou, Guangdong 510000 (CN)
(72) Inventor: JIA, Weihua, Guangzhou, Guangdong 510000 (CN); LIAO, Ying, Guangzhou, Guangdong 510000 (CN); DU, Yan, Guangzhou, Guangdong 510000 (CN)
(74) Representative: Chung, Hoi Kan
(86) International application number: PCT/CN2025/071495
(87) International publication number: WO 2025/161895

(57) **Abstract**

The present disclosure belongs to the field of biotechnology, and specifically relates to a methylated gene marker for early detection of endometrial carcinoma, a multiplex PCR detection kit, and Use Thereof. The methylated gene marker for early detection of endometrial carcinoma according to the present disclosure is selected from any at least one methylation region in any at least one of the target genes ADARB2, ADCYAP1, ADHFE1, C17orf64, CDO1, GAD2, GRM7, GYPC, KCNA1, KCNQ5, NETO1, RYR2, SORCS3, STX16, TCTEX1D1, TMEM101, and ZSCAN12. Diagnosis of endometrial carcinoma is performed by detecting the corresponding methylation regions, offering higher sensitivity and specificity, which is of significant importance for improving the diagnosis rate in high-risk populations for endometrial carcinoma, enabling early intervention and treatment, and reducing mortality.

## Description

### TECHNICAL FIELD

The present disclosure belongs to the field of biotechnology, and particularly relates to a methylated gene marker for early detection of endometrial carcinoma, a multiplex PCR detection kit and use thereof.

### BACKGROUND

Endometrial carcinoma is an epithelial malignant tumor occurring in the endometrium, also known as corpus uteri cancer, accounting for about 20-30% of gynecological malignancies. In recent years, the incidence of this tumor has gradually increased with a clear trend of affecting younger individuals; in some developed regions, its incidence has become the highest among gynecological malignancies.

Since endometrial carcinoma presents early symptoms such as irregular vaginal bleeding and vaginal discharge, about 70% of patients are diagnosed at an early stage confined to the corpus uteri. However, due to the non-specific nature of these symptoms, a significant proportion of patients are discovered only after losing the opportunity for early diagnosis. Early diagnosis is of great significance for endometrial carcinoma. On one hand, early diagnosis and treatment can significantly improve patient survival rates and quality of life; on the other hand, with the trend of younger patients, more individuals with endometrial carcinoma still have fertility needs, and early detection may provide the possibility for fertility-preserving treatment.

Currently, there are no recommended methods for routine screening of endometrial carcinoma. According to current guidelines for the diagnosis and treatment of endometrial carcinoma, clinical auxiliary diagnosis mainly relies on vaginal ultrasound and serum tumor markers such as CA125. However, these methods suffer from issues of insufficient sensitivity and/or specificity, making them unsuitable for population-based early routine screening of endometrial carcinoma. Endometrial cell collectors used for liquid-based cytology can screen for endometrial lesions in symptomatic and high-risk populations, but they are operator-dependent, have limited sensitivity, and involve somewhat invasive sampling. For early screening of endometrial carcinoma, more accurate and less invasive markers still need to be developed.

DNA methylation is a type of epigenetics, involving the addition of a methyl group to the 5-carbon atom of a cytosine base; this modification is typically associated with gene silencing. DNA methylation is a key epigenetic regulator of gene expression, often leading to defects in gene expression. Increased methylation of tumor suppressor genes is an early event in many tumors. Currently, methylated gene marker detection is widely recognized as a source of tumor screening markers. Moreover, using DNA methylation testing as a tumor screening method offers advantages of high sensitivity and specificity. However, there is currently a lack of detection technologies, methods, and products specifically for methylated gene testing in endometrial carcinoma. Therefore, there is an urgent need to provide a methylation-based early screening marker that can distinguish endometrial carcinoma from non-cancerous groups. This marker should be applicable to body fluid samples such as urine and blood, exfoliated cell collection samples like swabs, smears, and lavage fluid, as well as tumor tissue samples, and should be capable of accurately distinguishing endometrial carcinoma from other diseases/healthy individuals by eliminating background signal interference across various non-invasively collection samples.

### SUMMARY

To overcome the shortcomings and deficiencies of the prior art described above, the primary objective of the present disclosure is to provide a methylated gene marker for early detection of endometrial carcinoma.

The present disclosure has discovered novel methylated genes applicable for the diagnosis of endometrial carcinoma or its precancerous lesions: ADARB2, ADCYAP1, ADHFE1, C17orf64, CDO1, GAD2, GRM7, GYPC, KCNA1, KCNQ5, NETO1, RYR2, SORCS3, STX16, TCTEX1D1, TMEM101, and ZSCAN12. Detecting methylation of these genes for diagnosing endometrial carcinoma or its precancerous lesions offers higher sensitivity and specificity. The present disclosure provides new markers and diagnostic strategies for the diagnosis of endometrial carcinoma and its precancerous lesions.

Another object of the present disclosure is to provide a multiplex PCR detection kit for early screening and diagnosis of endometrial carcinoma.

A further object of the present disclosure is to provide use of the above methylated gene marker in formulation of a diagnostic reagent for endometrial carcinoma.

Yet another object of the present disclosure is to provide use of the above multiplex PCR detection kit in formulation of a diagnostic product for endometrial carcinoma.

The objects of the present disclosure are achieved through the following solutions:

A methylated gene marker for early detection of endometrial carcinoma, selected from at least one methylation region in at least one of the target genes ADARB2, ADCYAP1, ADHFE1, C17orf64, CDO1, GAD2, GRM7, GYPC, KCNA1, KCNQ5, NETO1, RYR2, SORCS3, STX16, TCTEX1D1, TMEM101, and ZSCAN12:
ADARB2 gene: chr10: 1779052-1779183, chr10: 1779756-1779910;
ADCYAP1 gene: chr18: 907522-907672, chr18: 908206-908385;
ADHFE1 gene: chr8: 67344466-67344599;
C17orf64 gene: chr17: 58498643-58498859, chr17: 58498879-58499081;
CDO1 gene: chr5: 115152259-115152414;
GAD2 gene: chr10: 26504914-26505063; chr10: 26506264-26506424; chr10: 26506907-26507068;
GRM7 gene: chr3: 6904068-6904274;
GYPC gene: chr2: 127413900-127414152;
KCNA1 gene: chr12: 5018675-5018893; chr12: 5019386-5019578;
KCNQ5 gene: chr6: 73331034-73331188; chr6: 73331252-73331411;
NETO1 gene: chr18: 70535858-70535996;
RYR2 gene: chr1: 237205894-237206034;
SORCS3 gene: chr10: 106400776-106400914, chr10: 106401432-106401587;
STX16 gene: chr20: 57225138-57225270;
TCTEX1D1 gene: chr1: 67218010-67218198;
TMEM101 gene: chr17: 42092213-42092341; and
ZSCAN12 gene: chr6: 28367209-28367379, chr6: 28367431-28367676.

By screening the types of genes related to endometrial carcinoma and the methylation region of each gene, the present disclosure ultimately identifies the above seventeen marker genes (or target genes) and their corresponding functionally optimal methylation regions. The methylation results of each marker gene can be used to determine interpretation thresholds, and the results of each methylation region can be applied for early detection of endometrial carcinoma with high accuracy, providing clinicians with auxiliary diagnostic references.

The present disclosure proposes a new marker that may be used for diagnosing endometrial carcinoma or its precancerous lesions, namely the above-mentioned methylated gene. Specifically, the present disclosure targets at least one of the target genes ADARB2, ADCYAP1, ADHFE1, C17orf64, CDO1, GAD2, GRM7, GYPC, KCNA1, KCNQ5, NETO1, RYR2, SORCS3, STX16, TCTEX1D1, TMEM101, and ZSCAN12, diagnosing endometrial carcinoma by detecting the corresponding methylation regions, with higher sensitivity and specificity, demonstrating potential and prospects as markers for diagnosing or screening endometrial carcinoma. Therefore, at least one of the above genes can serve as a biomarker for endometrial carcinoma, which is of significant importance for improving the diagnosis rate in high-risk populations, enabling early intervention and treatment, and reducing mortality.

Furthermore, endometrial carcinoma is diagnosed by detecting the methylation region corresponding to any at least one target gene in an in vitro detection sample. The sample may include body fluid samples such as urine and blood, exfoliated cell collection samples such as swabs, smears, and lavage fluid, as well as tumor tissue samples.

Moreover, when the sample is an exfoliated cell collection sample such as a swab, smear, or lavage fluid, targeting at least one of the above target genes and detecting the corresponding methylation regions for diagnosing endometrial carcinoma can achieve both sensitivity and specificity as high as 100%.

Furthermore, when the sample is urine, using a single one of the above target genes as a target and diagnosing by detecting the corresponding methylation region can still achieve a sensitivity of up to 91% and a specificity of up to 95%. Existing technologies mostly utilize detection from endometrial tissue and cervical smear cells, whereas the present disclosure requires only one molecular marker (i.e., one gene) for urine sample detection and can achieve high sensitivity and specificity, providing a simpler and more accurate method for the detection of endometrial carcinoma.

Furthermore, diagnosing endometrial carcinoma or precancerous lesions in the form of a panel of two or more of the above gene methylations can significantly improve sensitivity and specificity.

The present disclosure further provides a detection primer for early detection of endometrial carcinoma, used to detect a methylation status of the methylation region of the above marker genes, with the nucleotide sequences of the detection primers as follows:
ADARB2 gene:
   chr10: 1779052-1779183 corresponds to detection primers SEQ ID NO:1-2;
   chr10: 1779756-1779910 corresponds to detection primers SEQ ID NO:3-4;
ADCYAP1 gene:
   chr18: 907522-907672 corresponds to detection primers SEQ ID NO:5-6;
   chr18: 908206-908385 corresponds to detection primers SEQ ID NO:7-8;
ADHFE1 gene:
   chr8: 67344466-67344599 corresponds to detection primers SEQ ID NO:9-10;
C17orf64 gene:
   chr17: 58498643-58498859 corresponds to detection primers SEQ ID NO:11-12;
   chr17: 58498879-58499081 corresponds to detection primers SEQ ID NO:13-14;
CDO1 gene:
   chr5: 115152259-115152414 corresponds to detection primers SEQ ID NO:15-16;
GAD2 gene:
   chr10: 26504914-26505063 corresponds to detection primers SEQ ID NO:17-18;
   chr10: 26506264-26506424 corresponds to detection primers SEQ ID NO:19-20;
   chr10: 26506907-26507068 corresponds to detection primers SEQ ID NO:21-22;
GRM7 gene:
   chr3: 6904068-6904274 corresponds to detection primers SEQ ID NO:23-24;
GYPC gene:
   chr2: 127413900-127414152 corresponds to detection primers SEQ ID NO:25-26;
KCNA1 gene:
   chr12: 5018675-5018893 corresponds to detection primers SEQ ID NO:27-28;
   chr12: 5019386-5019578 corresponds to detection primers SEQ ID NO:29-30;
KCNQ5 gene:
   chr6: 73331034-73331188 corresponds to detection primers SEQ ID NO:31-32;
   chr6: 73331252-73331411 corresponds to detection primers SEQ ID NO:33-34;
NETO1 gene:
   chr18: 70535858-70535996 corresponds to detection primers SEQ ID NO:35-36;
RYR2 gene:
   chr1: 237205894-237206034 corresponds to detection primers SEQ ID NO:37-38;
SORCS3 gene:
   chr10: 106400776-106400914 corresponds to detection primers SEQ ID NO:39-40;
   chr10: 106401432-106401587 corresponds to detection primers SEQ ID NO:41-42;
STX16 gene:
   chr20: 57225138-57225270 corresponds to detection primers SEQ ID NO:43-44;
TCTEX1D1 gene:
   chr1: 67218010-67218198 corresponds to detection primers SEQ ID NO:45-46;
TMEM101 gene:
   chr17: 42092213-42092341 corresponds to detection primers SEQ ID NO:47-48;
ZSCAN12 gene:
   chr6: 28367209-28367379 corresponds to detection primers SEQ ID NO:49-50;
   chr6: 28367431-28367676 corresponds to detection primers SEQ ID NO:51-52.

The present disclosure further provides a multiplex PCR detection kit for early screening and diagnosis of endometrial carcinoma, including a reagent for detecting methylation of a target gens;
the target gene is selected from at least one of the following: ADARB2, ADCYAP1, ADHFE1, C17orf64, CDO1, GAD2, GRM7, GYPC, KCNA1, KCNQ5, NETO1, RYR2, SORCS3, STX16, TCTEX1D1, TMEM101, and ZSCAN12.

The reagent includes at least one of the detection primers described above.

Further, the workflow of the multiplex PCR detection kit may be:
(1) extracting DNA from a sample to be tested and performing conversion to obtain modified DNA;
(2) using the multiplex PCR detection kit to detect the modified DNA, with the detection procedure including two rounds of PCR reactions; the primer for the first round of multiplex PCR includes two parts: a target region amplification primer fragment, and a sequencing ligation primer fragment including a forward sequencing primer and a reverse sequencing primer; the primer for the second round of multiplex PCR include a fragment complementary to the sequencing primer ligation fragment in the first round PCR primer and another fragment containing a sequencing adapter.

In the above workflow, the conversion refers to achieving DNA methylation conversion; the reagent used may be conventional methylation conversion reagents, such as but not limited to bisulfite, bisulfite hydride, or hydrazine salt treatment, and enzymatic conversion.

Further, next-generation sequencing is performed on the amplification product obtained after the two rounds of PCR reactions.

Further, a length of the target region amplification primer fragment is 26-38bp.

Further, the forward detection primer obtained from the first round of multiplex PCR includes a forward sequencing primer + NNNNNNNN + a forward amplification primer, with a length of 63-75bp; the reverse detection primer includes a reverse sequencing primer + NNNNNNNN + a reverse amplification primer, with a length of 69-72bp; adjustments may also be made according to the testing instrument.

Next-generation sequencing may be directly performed using an Illumina sequencer (sequencing modes such as PE150, PE250, SE150, SE250 are all applicable).

The present disclosure further provides use of the above methylated gene marker in preparation of a product for diagnosing endometrial carcinoma.

The present disclosure further provides use of the above multiplex PCR detection kit in preparation of the product for diagnosing endometrial carcinoma.

The diagnostic products include any one of a kit, a formulation, and a chip.

Compared to the prior art, the present disclosure has the following advantages and beneficial effects:
By screening the types of genes associated with endometrial carcinoma and the methylation region of each gene, the present disclosure ultimately selects the aforementioned seventeen marker genes (or target genes) and their corresponding functionally optimal methylation regions, which can significantly improve the sensitivity and specificity for early detection of endometrial carcinoma. The methylated gene markers of the present disclosure can be applied to body fluid samples such as urine and blood, exfoliated cell collection samples such as swabs, smears, and lavage fluid, as well as tumor tissue samples, and can eliminate background signal interference across various sample types to accurately distinguish detection targets for endometrial carcinoma from other diseases/healthy individuals. For non-invasively collected urine samples, even with a low DNA template concentration, the methylated gene markers of the present disclosure can achieve excellent detection sensitivity and specificity, reducing unnecessary traumatic sampling. With advantages such as non-invasive sampling, higher sensitivity and specificity, it not only optimizes the allocation of medical resources, improves subject acceptance, enhances the screening experience, but also ensures the accuracy of results, thereby aiding in achieving the goal of early detection, early diagnosis, and early treatment of endometrial carcinoma.

### BRIEF DESCRIPTION OF DRAWINGS

To more clearly illustrate the technical solutions of the embodiments of the present disclosure, the following briefly introduces the drawings required for the embodiments. It should be understood that the following drawings only show certain embodiments of the present disclosure and therefore should not be considered as limiting the scope. For those of ordinary skill in the art, other related drawings can be obtained based on these drawings without creative effort.
FIG. 1 is a flowchart of the kit detection for multiplex PCR amplification methylation sequencing used for sequencing multiple methylation regions.
FIG. 2 is a flowchart of library construction for multiplex PCR amplification methylation sequencing.
FIG. 3 is a diagram showing the effect of DNA fragmentation on multiplex targeted amplicon methylation sequencing.
FIG. 4 is a diagram showing the effect of sample input DNA amount on multiplex targeted amplicon methylation sequencing.
FIG. 5 shows the resequencing consistency results between multiplex targeted amplicon methylation sequencing and DNA methylation chip technology.
FIG. 6 is a diagram of the methylation rate levels of gene methylation markers in cancerous tissues, adjacent tissues, and non-cancerous normal tissues of endometrial carcinoma.
FIG. 7 is a diagram of the changes in methylation rates of gene methylation markers in paired pre-operative and post-operative samples of endometrial carcinoma.

### DETAILED DESCRIPTION OF THE EMBODIMENTS

The present disclosure is further described in detail below with reference to embodiments, but the embodiments of the present disclosure are not limited thereto. Materials involved in the following embodiments, unless otherwise specified, can be obtained from commercial sources. The methods, unless otherwise specified, are conventional methods.

### Example 1: Screening of methylated gene markers for endometrial carcinoma

In the present disclosure, the TCGA public database was utilized to obtain 450K methylation chip data from 524 cancer tissues and 34 adjacent tissues of endometrial carcinoma, screening for sites significantly hypermethylated in endometrial carcinoma tissues; then the CHAMP.DMP method was used for differential site screening, with the criteria of FDR-adjusted *P* value <0.05 and (average methylation rate of cancer tissue - average methylation rate of adjacent tissue) >0.35, resulting in a total of 8730 differential sites screened.

Since sampling methods such as urine, pap smear, vaginal swab, and blood inevitably include components like white blood cells or urothelial cells in addition to tumor-derived cells or DNA, the incorporation of these non-target exogenous DNAs may interfere with the detection of target markers as background signals. Therefore, careful handling of background signal interference is required when screening markers. Considering the above, the present disclosure further incorporated 450K methylation chip data from 78 female urinary tract adjacent tissues and 338 peripheral blood leukocytes in the TCGA and GEO databases, performed CHAMP.DMP differential testing with data from 425 endometrial carcinoma tissues.

Sites meeting all the following conditions were included in the screening range:
a) FDR-adjusted *P* value <0.05 in both differential tests; (2) (average methylation rate of cancer tissue - average methylation rate of urinary tract adjacent tissue) >0; (3) (average methylation rate of cancer tissue - average methylation rate of peripheral blood leukocytes) >0; (4) average methylation rate of urinary tract adjacent tissue <0.1; and (5) average methylation rate of peripheral blood leukocytes <0.1. Sites satisfying the above requirements were intersected with the differential sites from the endometrial carcinoma/adjacent tissue differential screening, resulting in 1464 sites included in the next screening step.

To further evaluate the methylation levels of the aforementioned 1,464 candidate sites in actual samples, this disclosure performed Illumina Epic 850k chip detection on exfoliated cell samples from 12 endometrial carcinoma patients and 15 controls collected at the Sun Yat-sen University Cancer Center and the First People's Hospital of Foshan. This chip was an upgraded version of a 450K chip, retaining most of the 450K chip sites while adding nearly 400,000 new methylation sites. Ultimately, 17 genes were selected, including C17orf64, CDO1, KCNQ5, STX16, ZSCAN12, KCNA1, ADARB2, SORCS3, GAD2, RYR2, GRM7, ADCYAP1, NETO1, GYPC, TMEM101, ADHFE1, and TCTEX1D1. The methylation sites with the best diagnostic performance for endometrial carcinoma among these genes and their performance information are shown in Table 1.

The areas under the curve for the optimal sites of the 17 genes ranged from 0.867 to 0.983. Among them, 7 genes including C17orf64, CDO1, KCNQ5, STX16, ZSCAN12, KCNA1, and ADARB2 had AUCs greater than 0.98; 7 genes including SORCS3, GAD2, RYR2, GRM7, ADCYAP1, NETO1, and GYPC had AUCs between 0.90 and 0.95. Different genes exhibited distinct advantages in detection sensitivity and specificity: the sensitivity of CDO1, KCNA1, and TCTEX1D1 genes could reach 100%, while the specificity of C17orf64, KCNQ5, STX16, and ZSCAN12 genes could reach 100%. Except for GRM7 and GAD2, the sensitivity of these 17 gene methylation markers was above 80%; except for TCTEX1D1, the specificity was above 80%. Overall, when specificity was 100%, the sensitivity of C17orf64, KCNQ5, STX16, and ZSCAN12 genes could reach 91.7%; when sensitivity was 100%, the specificity of KCNA1 gene could reach 93.3%. These genes demonstrated excellent discriminatory ability between endometrial carcinoma and controls.

Furthermore, sites with strong discriminatory ability clustered around the optimal sites. Within 500 bp upstream and downstream of the best site, the aforementioned genes had 1 to 8 sites with AUC greater than 0.8, with 14 genes having at least 3 or more sites with AUC above 0.8. Genes with fewer than 3 sites with AUC>0.8 were likely due to lower probe coverage density and fewer detection sites for these genes in the Epic 850k chip design. The clustering of multiple sites further indicates the certainty of using methylation of these genes as diagnostic markers for endometrial carcinoma and is beneficial for the development of methylation site detection methods. The results show that the 17 genes of this disclosure possess excellent discriminatory ability between endometrial carcinoma and control populations.

**Table 1**

| Gene Name | Best Predictive Methylation Site | Location of Best Site (hg19) | Sensitivity (%) | Specificity (%) | AUC | A* |
|---|---|---|---|---|---|---|
| C17orf64 | cg06381367 | CHR17: 58499044 | 91.7 | 100.0 | 0.983 | 4 |
| CDO1 | cg23180938 | CHR5: 115152485 | 100.0 | 86.7 | 0.983 | 7 |
| KCNQ5 | cg05447008 | CHR6: 73331114 | 91.7 | 100.0 | 0.983 | 4 |
| STX16 | cg13186228 | CHR20: 57225195 | 91.7 | 100.0 | 0.983 | 1 |
| ZSCAN12 | cg25060829 | CHR6: 28367571 | 91.7 | 100.0 | 0.983 | 6 |
| KCNA1 | cg12411513 | CHR12: 5019475 | 100.0 | 93.3 | 0.961 | 6 |
| ADARB2 | cg23684973 | CHR10: 1780003 | 91.7 | 86.7 | 0.956 | 5 |
| SORCS3 | cg01874697 | CHR10: 106401517 | 83.3 | 93.3 | 0.944 | 3 |
| GAD2 | cg12527175 | CHR10: 26504950 | 75.0 | 93.3 | 0.928 | 4 |
| RYR2 | cg07914084 | CHR1: 237205999 | 83.3 | 93.3 | 0.922 | 1 |
| GRM7 | cg16415870 | CHR3: 6904261 | 75.0 | 93.3 | 0.917 | 3 |
| ADCYAP1 | cg18319029 | CHR18: 904920 | 83.3 | 93.3 | 0.911 | 8 |
| NETO1 | cg21922574 | CHR18: 70535925 | 83.3 | 86.7 | 0.906 | 1 |
| GYPC | cg15975865 | CHR2: 127413831 | 91.7 | 86.7 | 0.906 | 3 |
| TMEM101 | cg24638647 | CHR17: 42092247 | 91.7 | 86.7 | 0.888 | 13 |
| ADHFE1 | cg08090772 | CHR8: 67344640 | 91.7 | 80.0 | 0.878 | 5 |
| TCTEX1D1 | cg09318375 | CHR1: 67218090 | 100.0 | 66.7 | 0.867 | 3 |

| | | | | | | |
|---|---|---|---|---|---|---|
| Note: A* indicates the number of sites with AUC > 0.8 within 500 bp upstream and downstream of the optimal site. | | | | | | |

### Example 2: Construction of multiplex PCR amplification methylation sequencing technology for detecting methylation signals of markers

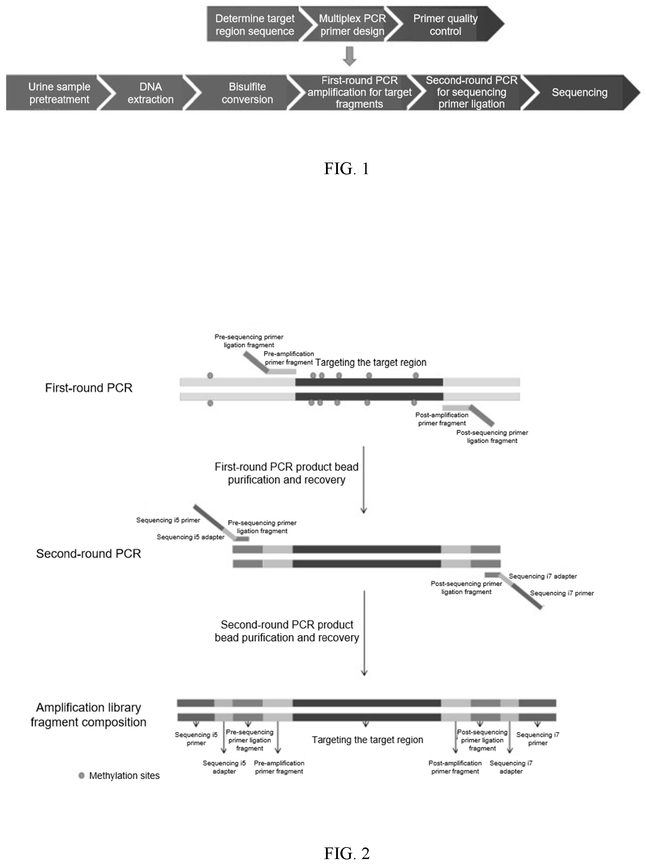

For the 17 genes screened in Example 1, a DNA amplicon-targeted methylation sequencing method was established. Targeted PCR amplification was performed on bisulfite-converted DNA, and detection was carried out via next-generation sequencing to obtain the methylation levels of candidate markers in the samples. The flowchart is shown in FIG. 1. Specific steps are as follows:

### 2.1 Design of multiplex PCR primers for target regions

Determination of target regions: Sites on CpG islands often appear consecutively. To determine the optimal sequence amplification range, the present disclosure first selected the best amplification region gene by gene for the aforementioned 17 candidate differentially methylated sites. Using the site with the highest area under the receiver operating characteristic curve (AUC) within the gene as the center, the methylation rates of all methylation sites included in a 850k chip for that gene in exfoliated cell and endometrial carcinoma tissue data were extracted. A 150-350 bp region with the densest methylation sites and the most significant difference between exfoliated cells and tissues was then selected for subsequent primer design.

Design of PCR primers for target regions: DNA methylation-specific PCR primer design software such as MethPrimer was used to design primers for each sequence. The designed primers were paired, and compatibility between primer pairs was compared to minimize issues such as primer dimers and hairpin structures among multiple primer pairs, thereby improving primer success rates. After repeated optimization, 26 primer pairs were designed for the 17 target genes for multiplex PCR detection. Primer sequences, chromosomal physical positions of the amplified regions, and information on the target genes are listed in Table 2. Here, "F" denotes a forward amplification primer, and "R" denotes a reverse amplification primer.

**Table 2**

| Gene detection panel name | Primer sequence name | Nucleotide sequence (5'-3') | SEQ ID NO |
|---|---|---|---|
| ADARB2 gene primer-probe panel 1 (ADARB2_1) | ADARB2_F_1 | TTTTGGGTAAGAGGAGGTTTTAGATA | 1 |
| | ADARB2_R_1 | CTCTCATAAAATCTCCRAACTTATCC | 2 |
| ADARB2 gene primer-probe panel 2 (ADARB2_2) | ADARB2_F_2 | GTAGYGTTTAATTAGATTTAATTATAATT GTTTTTGG | 3 |
| | ADARB2_R_2 | TTCCCCRAAAACAACCTCCCTCCAAC | 4 |
| ADCYAP1 gene primer-probe panel 1 (ADCYAP1_1) | ADCYAP1_F_1 | GTTTTGGGAGAAGAGATAATTTTTAGYGG AGGATT | 5 |
| | ADCYAP1_R_1 | CTCTTCCTCTAACCTACRAAATAACC | 6 |
| ADCYAP1 gene primer-probe panel 2 (ADCYAP1_2) | ADCYAP1_F_2 | TTAGYGGTTATTTGGGGATAGAAATAG | 7 |
| | ADCYAP1_R_2 | AATCCTTCCACAAACTCTTACCCCAC | 8 |
| ADHFE1 gene primer-probe panel 1 (ADHFE1_1) | ADHFE1_F_1 | AGTTGATAAGTAAGGAGATTTAAGGTAG | 9 |
| | ADHFE1_R_1 | CATTTTCCCACRAAATAAAAACCAAAAC | 10 |
| C17orf64 gene primer-probe panel 1 (C17orf64_1) | C17orf64_F_1 | TGTTGAATATGTTGGTATAGAGGATAAG | 11 |
| | C17orf64_R_1 | CCCCACTCAATATAACCCTATTCTTC | 12 |
| | C17orf64_F_2 | GAGGATAYGAAGGAAGGTTYGGGAGAAG | 13 |
| C17orf64 gene primer-probe panel 2 (C17orf64_2) | C17orf64_R_2 | TTAACCRAACAAAACCRAAAAATACTCA AAAAAC | 14 |
| CDO1 gene primer-probe panel 1 (CDO1_1) | CDO1_F_1 | GTAGAGATAGTAAGAGATTTATTTTTAGG | 15 |
| | CDO1_R_1 | CRTTAATACATTCCTAATAACTCCAAAC | 16 |
| GAD2 gene primer-probe panel 1 (GAD2_1) | GAD2_F_1 | TTAGGTTTTATTAAGGYGTTGAAATGAG | 17 |
| | GAD2_R_1 | TTCCTCCAAACAAATACTAAAATCACTA | 18 |
| GAD2 gene primer-probe panel 2 (GAD2_2) | GAD2_F_2 | AGAGGAATTTGTTTGAGTYGATTGGTA | 19 |
| | GAD2_R_2 | TTCCTCCTAAAAACCRCTCAAAACTC | 20 |
| GAD2 gene primer-probe panel 2 (GAD2_3) | GAD2_F_3 | TTTTTATGTAATAGGTAAAGATTTAGYGG GGAGTT | 21 |
| | GAD2_R_3 | CRCTTTTATCTAAAAATTCTAACAAATCCC | 22 |
| GRM7 gene primer-probe panel 1 (GRM7_1) | GRM7_F_1 | GATAGTTTTAAGGAGGTATTTTTTAAATY GAGGGTTT | 23 |
| | GRM7 R 1 | CAAAATATTACCCRAAACAAACCAACC | 24 |
| GRM7 gene primer-probe panel 1 (GRM7_1) | GRM7_F_1 | TYGTGGGGAAGGGTTTTGGGGATTTATTG | 25 |
| | GRM7_R_1 | AAACRAACTAACRACTTCTCCAACTC | 26 |
| KCNA1 gene primer-probe panel 1 (KCNA1_1) | KCNA1_F_1 | GATYGAAGAGGGTTATTGTGGGATTG | 27 |
| | KCNA1_R_1 | CACAACTTTTTCCTCTCCTACCAAAA | 28 |
| KCNA1 gene primer-probe panel 2 (KCNA1_2) | KCNA1_F_2 | TAGGTTGTTGGTAATYGGTGAGAATG | 29 |
| | KCNA1_R_2 | CRACAAATCRTCTTTACAATACCCTAAC | 30 |
| KCNQ5 gene primer-probe panel 1 (KCNQ5_1) | KCNQ5_F_1 | ATTGGAAAGGATGTTTAGTTATTYGYGAG TTTGT | 31 |
| | KCNQ5_R_1 | TCCTCAACAATTCAATAAACAAACCC | 32 |
| KCNQ5 gene primer-probe panel 2 (KCNQ5_2) | KCNQ5_F_2 | GGTTAGATAATATTTGGAAGGTAGGG | 33 |
| | KCNQ5_R_2 | CTATCCRAAAAATAAAATCAACCTTATAT CC | 34 |
| NETO1 gene primer-probe panel 1 | NETO1_F_1 | GTAAAAGTTTYGYGGTTGAGTATTGG | 35 |
| (NETO1_1) | NETO1_R_1 | CRAAAATCCRTATATACCTTAAAATAATT TTAAATACC | 36 |
| RYR2 gene primer-probe panel 1 (RYR2_1) | RYR2_F_1 | TGTTAGGGGAAGGGGGYGTTAGGGTA | 37 |
| | RYR2_R_1 | ACACCTACACACACTTAATAATACCC | 38 |
| SORCS3 gene primer-probe panel 1 (SORCS3_1) | SORCS3_F_1 | GTTTAGTGGGGAGGATTGGGGGGAAT | 39 |
| | SORCS3_R_1 | CTCTCCACTCTTCCCTCCTAACTCCC | 40 |
| SORCS3 gene primer-probe panel 2 (SORCS3_2) | SORCS3_F_2 | GTYGGGGTATTTTAGTTTTTGTTAAGTT | 41 |
| | SORCS3_R_2 | CCTCCTCCCRAAAACCCTTAACAAAA | 42 |
| STX16 gene primer-probe panel 1 (STX16_1) | STX16_F_1 | GTGTAAAGTTTGTTTTAYGTAGAAATAAA GG | 43 |
| | STX16_R_1 | CCTATACAAAACCRACTTCCCCTTAC | 44 |
| TCTEX1D1 gene primer-probe panel 1 (TCTEX1D1_1) | TCTEX1D1_F_1 | AGAGTGATTTTTAGTGGGTAGTGAAG | 45 |
| | TCTEX1D1_R_1 | TCCCRCRTCCCAAACTTCATTCAACC | 46 |
| TMEM101 gene primer-probe panel 1 (TMEM101_1) | TMEM101_F_3 | TATGAGTTGGTTGAAGTAGTTTTAAAAG | 47 |
| | TMEM101_R_3 | AACRAACTACCCTTTCCCAAAATAAC | 48 |
| ZSCAN12 gene primer-probe panel 1 (ZSCAN12_1) | ZSCAN12_F_1 | GAAGGTATGGGTTTTATAGTTTGGAA | 49 |
| | ZSCAN12_R_1 | TTCCAACTACTAAACCCCTCAAACCC | 50 |
| ZSCAN12 gene primer-probe panel 2 (ZSCAN12_2) | ZSCAN12_F_2 | TATTTGYGGTTTTTAGGGTTAGAAGAG | 51 |
| | ZSCAN12_R_2 | CTAAAAACCATCAACCTTCCTCTCAC | 52 |

### 2.2 Two rounds of PCR amplification to construct an amplicon library of the target fragment for sequencing

Urine sample pre-treatment and DNA extraction were performed using the ZYMO Quick-DNA^{™} Urine Kit, and DNA bisulfite conversion was performed using the ZYMO EZ DNA Methylation-Gold Kit. For bisulfite-converted DNA samples, this disclosure established a multiplex PCR primer system targeting 26 fragments across 17 target genes, capable of cumulatively detecting 280 methylation sites. The method consists of two rounds of PCR: the primers for the first multiplex PCR are divided into two parts-target region amplification primer fragments of 26-38 bp and sequencing ligation primer fragments of 29 bp (forward primer) and 26 bp (reverse primer); the resulting first-round multiplex forward PCR primers are 63-75 bp in length, and the reverse PCR primers are 69-72 bp in length. The primers for the second multiplex PCR include a complementary fragment to the sequencing ligation fragment in the first-round PCR primers and another primer fragment containing sequencing adapters. The amplification products from the two rounds of PCR can be directly used for next-generation sequencing on Illumina sequencers (compatible with sequencing modes such as PE150, PE250, SE150, SE250). The design principle of the two-round PCR primers is illustrated in FIG. 2.

### Specific steps are as follows:

### (1) First-round PCR amplification

The first-round PCR was a multiplex PCR amplification system, with its amplification primers divided into two parts: {sequencing primer fragment + 8 free bases + amplification primer fragment}. This kit covered the detection of 28 methylation regions, including the 17 genes described in this application. The sequences of the amplification primer fragments for each amplification region are listed in Table 2. Basic information of each gene's methylation detection region, reference genome positions of the detection sequences, detection sequence lengths, and reference genome sequences of the detection regions are provided in Table 3.

**Table 3**

| Genetic Testing Panel | Genomic Region Tested | Test Region Length (bp) | Reference Genome Sequence of Test Region |
|---|---|---|---|
| ADARB2_ 1 | chr10: 1779052-1779183 | 132 | ctctgggcaagaggaggccccagacatttccacgggatctgaactcccgagcgccctgc acggagcagccatcccgccagcaccccaaagtgaagctgctcacaacggacaagcccg gagaccccatgagag |
| ADARB2_ 2 | chr10: 1779756-1779910 | 155 | gcagcgtctaattagatccaatcacaactgctttctggctctgacatcagcagcagcaccag ccgcaccgccgcctggccgcttgcaaactcagctcccgtttggacggtgaagctcccagg accggcggctggagggaggctgctcccggggaa |
| ADCYAP1 _1 | chr18: 907522-907672 | 151 | gtcctgggagaagagacaattctcagcggaggacttttatcacctgtgaaaatccgcgcga gccccttactttggatcctcgccgagctggggaggaacttgcactgaccacaccttctgtcc ccggccaccccgcaggccagaggaagag |
| ADCYAP1 _2 | chr18: 908206-908385 | 180 | ctagcggccacctggggacagaaacagtgaccctgggcgcgcactttgcctccccgtta gagatgtcgcccacgggatccttaacgaggcctaccgcaaagtgctggaccagctgtcc gccgggaagcacctgcagtcgctcgtggcccggggcgtggggtaagagtttgtggaag gatt |
| ADHFE1_1 | chr8: 67344466-67344599 | 134 | agttgataagtaaggagatttaaggcagaacccgaggcctacggagcagttaccttctacg gcaatttcaagggtggatggtgcgagcgccgctggggcagctggcgttctggttcttactc cgtgggaaaatg |
| C17orf64_1 | chr17: 58498643-58498859 | 217 | tgctgaacatgctggcatagaggacaagttaaaaagtttctggactcaaagggtgctcctcc aaagctccgggcctccgggctacccctacttgaagccgctatgcccctctcctgtgcccga ggtggccgctgggtggcaggggaggcccgggccgacggcggtcccaccgcactgtcg cgaaggcaggcgaagaacagggccacactgagtgggg |
| C17orf64_2 | chr17: 58498879-58499081 | 203 | gaggacacgaaggaaggtccgggagaaggaattcctagtttcagggaagaaaaggtgg ctcgagtgaggaaactgcgggggcaacggtctctcgtcgccgggaccccgccgtgtgcc gcccagggacgcgagggggcgctgctgccgcgccaggggcgagggtcccggggcct cctgagcacctcccggccctgcccggccaa |
| CDO1_1 | chr5: 11515225 9-115152414 | 156 | gcagagacagcaagagacccacccccaggcccctggcagcgcagtggatccgggatc gctggagacgcggtgcacacacaaatcaggttcagatctgtggggttcatcctcccgggc cccttttaagcgcttggagtcactaggaatgtaccaacg |
| GAD2_1 | chr10: 26504914-26505063 | 150 | tcaggttctaccaaggcgctgaaatgagcccatcagcgggtaggagcccttcccccgccg tcccctccccaggctcgtgaacggcgcctgatgcccgcccggggcgcgagctctcgag gtcgcagtgacctcagcacctgcttggaggaa |
| GAD2_2 | chr10: 26506264-26506424 | 161 | agaggaatttgcctgagccgattggcacctggaagggcagcggtgtgggcttctcctcgg gaaacagataaaggaaatgagggctggcccgggccgccagcctcccgcttgccttctgc acctgccggcctcaccgagtcctgagcggcccccaggaggaa |
| GAD2_3 | chr10: 26506907-26507068 | 162 | tctccatgcaacaggtaaagactcagcggggagccccgggcgcccctgcccctctctct gccccgcccaccgcggccggtgcgggtccggcgctgagagccggacaggggcgtcg aggtggcagcggaggcgggacctgccagaatcttcagataaaagcg |
| GRM7_1 | chr3: 6904068-6904274 | 207 | gacagccttaaggaggcacttcttaaatcgagggctccgtcttaacctagatgtggatgtta agtccgcatctccctcgggctgatttcccgacctgtagccagcctccgcgagagcccagg gcgcgaggtgctgggaccttcctcaggtggagagatgctgcccgcagacgtgacccccg gttggtttgctccgggcaatattttg |
| GYPC_1 | chr2: 12741390 0-127414152 | 253 | ccgtggggaagggtcctggggacccactggaggccgcggcccgcagcagccagggg ccgagccacggccacggacgccctggtgtcccggtccgtgccgggcctccaggcgga ggaggcgtccgctgggctcagatccccgactccagccccggttccccggcgcctgggct gcgcggagtccctgtcccgcgtcccggacccctacgcgcagcctccacgcgctccgag ctggagaagccgccagcccgccc |
| KCNA1_1 | chr12: 5018675-5018893 | 219 | gatcgaagagggttactgtgggactgcacaaaagcaaacccgtcggaggagttttgccag aaacaccaccgcctgcattgcgtcggacctgaccatttccaatgtgaaattcccggggaag gtcgcgagccgctaggggccgttcgtgggcggggcggcgggccacaggggaagtaga gttagcggtcggcttttctggtaggagaggaaaaagctgtg |
| KCNA1_2 | chr12: 5019386-5019578 | 193 | caggttgctggcaaccggtgagaatgggggtagggaaggaacattttcgccgtagctgct ccgtaaagcgattgtccaactgagaggggcgtcggacgagtggaccagggcggcgagt ttgcccggcgcgtctcggatgctgctgcggcggccgccgcggctcccgccagggcact gcaaagacgacctgccg |
| KCNQ5_1 | chr6: 73331034-73331188 | 155 | actggaaaggatgttcagttactcgcgagcttgtctgatactgggatggaaagtcacagtcc gcaggctggagtgaggcgcgggaagatgcctggtccttgcctcgcggacttggcagccg cgtcctgcgggtctgtccactgaactgctgagga |
| KCNQ5_2 | chr6: 73331252-73331411 | 160 | ggctagataatacctggaaggcagggcagggctgttcccgctctcaccccttgcggaagg atggcaggatccggcgcagcgacgtagcagcgggagggcacagcgacctgcatctcca gtttctcctcaggacacaaggctgacttcaccttccggacag |
| NETO1_1 | chr18: 70535858-70535996 | 139 | gtaaaagtttcgcggttgagcactgggcctgatgtccagtccccccaccaaattactcctgc aaagacgcgggcttcttgcaattgagccccccacctcgaggtatttaaaaccaccccaag gcacacacggacccccg |
| RYR2_1 | chr1: 23720589 4-237206034 | 141 | tgtcaggggaagggggcgtcagggcatccactagcggggtccgggcagagtgacagc gggcagcggggactcgcgggcggggcgagggggtgccccctgaggatgcgggagg agcgggcatcaccaagtgtgtgcaggtgt |
| SORCS3_1 | chr10: 10640077 6-106400914 | 139 | gctcagtggggaggattgggggaatctgattttaagaaagaaagcaccgaagggcaa ttattaattttcctcgggtttggaatcaccctctggacaagagaacgggcgagcgggagcta ggagggaagagtggagag |
| SORCS3_2 | chr10: 10640143 2-106401587 | 156 | gccggggcatcccagctcctgccaagcttggcggcgcgaggaggagtcgccgggcgc agccccccaatcacccaggaacgcggggacgcctgggccactgctccggccgatggttc cagaggaagccgtccccttgctaagggttcccgggaggagg |
| STX16_1 | chr20: 57225138-57225270 | 133 | gtgtaaagtttgttccacgcagaaacaaaggacgcgtgggggccgcttctggggcctcgg tcctttgttggaaccccgcaccgcagcccggcccgcagcctcgccccgcaaggggaag ccggccctgcacagg |
| TCTEX1D 1_1 | chr1: 67218010-6721819 8 | 189 | agagtgacccccagtgggcagtgaagatgagatcctctacatccttagggcagctcagag ggccactcagcggccaagtccggccggggagggccgagggctctgtttgaggtcaccc tggagaccggcctcagagtccagggagagtgcgcgggcggccgccggctgaatgaag cctgggacgcggga |
| TMEM101 _1 | chr17: 42092213-42092341 | 129 | catgagctggctgaagcagccccaaaaggggcagcgtgtgagcagcaccgaacccaac tgcatgatcagctgcaacatccaccgtctcgaacctatcttcgacgccatcttgggaaagg gcagtccgct |
| ZSCAN12_ 1 | chr6: 28367209-28367379 | 171 | gaaggtatgggccttacagcctggaattgctgcagagactgggagccaaaccctcatcct aatgtcccaacgcactttggggagggagagcttcccgcggatccgcctgggtttgggagg accggggtccactctgcaccggccagggcctgagggcccagcagctggaa |
| ZSCAN12_ 2 | chr6: 28367431-28367676 | 246 | cacctgcggcccccagggccagaagagaactccctcctacggagcgaaggcaagagg cctcgaacccttttgggacccggaacccatcaaaagtgacccacaaaggccggaagcgg ccacgggggggtctaagaaccagcccgcgcggggcgcacttccgcggccgctctaggaa gggagcgaaaggggctttcaactcggtagtgtttccgcgcgtctacgtgagaggaaggtt gatggctttcag |

The design rationale for the primer sequences of the first-round multiplex PCR primers is shown in Table 4. The overall primer composition was "a sequencing primer + NNNNNNNN + an amplification primer". The forward sequencing primer sequence is 5'-AATGATACGGCGACCACCGAGATCTACAC-3'; eight variable bases "NNNNNNNN" were inserted in the middle to serve as a barcode sequence for distinguishing sample information in the sequenced reads; this was then linked to the forward methylation-specific amplification primer for each gene, detailed as the F primers in Table 2. The reverse sequencing primer sequence was 5'-CAAGCAGAAGACGGCATACGAGATTC-3'; eight variable bases "NNNNNNNN" were inserted in the middle as a barcode to distinguish sample information in the sequenced reads; this was then linked to the reverse methylation-specific amplification primer for each gene, detailed as the R primers in Table 2. In this disclosure, the first-round multiplex PCR primers for each amplification region were mixed in equal proportions to serve as the PCR primers. Using bisulfite-converted DNA as the template DNA, amplification was performed with KAPA HiFi Mix as the DNA polymerase. The number of amplification cycles was determined based on the quality and concentration of the DNA template, controlled within 15-35 cycles. The PCR reaction system composition was (per well): 2 µL 5x KAPA HiFi buffer; 0.3 µL 10 mM dNTPs; *0.5 µL DMSO*; 0.2 µL KAPA HiFi Polymerase; 1 µL forward sequencing primer (5 µM); 1 µL reaction sequencing primer (5 µM); 3 µL nuclease-free water; 2 µL converted DNA. PCR reaction conditions: first 95°C-5min; then 98°C-20s, 50-60°C-30s, 72°C-1min, 15-35 cycles; finally 4°C hold.

**Table 4**

| Primer type | Primer composition | Details |
|---|---|---|
| First round multiplex PCR forward primer | Overall composition | Forward sequencing primer +NNNNNNNN + forward amplification primer |
| | Forward sequencing primer | 5'-AATGATACGGCGACCACCGAGATCTACAC-3' |
| | Forward amplification primer | Forward amplification primers for different amplification regions are listed in Table 2 |
| First round multiplex PCR reverse primer | Overall composition | reverse sequencing primer +NNNNNNNN + reverse amplification primer |
| | Reverse sequencing primer | 5'-CAAGCAGAAGACGGCATACGAGATTC-3' |
| | Reverse amplification primer | Reverse amplification primers for different amplification regions are listed in Table 2 |

### (2) Second-round PCR amplification and purification

The first-round PCR amplification products were purified using AMPure XP beads; the resulting nucleic acid served as the template DNA for the second-round PCR. A second-round amplification was performed using KAPA HiFi Mix as the DNA polymerase. The annealing temperature and extension time were determined based on the specific product, with the number of cycles kept within 10-20 cycles. The forward primer sequence for the second-round amplification was CAAGCAGAAGACGGCATACGAGATNNNNNNNNGTCTCGTGGGCTCGG, and the reverse primer sequence was AATGATACGGCGACCACCGAGATCTACACNNNNNNNNTCGTCGGCAGCGTC, where "NNNNNNNN" represents the sequencer index sequence. Note that the first-round PCR sequencing primers and second-round sequencing primers can be modified according to the specific requirements of different sequencer brands and specifications; the sequencing primers provided in this disclosure are merely one example. Using other sequencing primers that comply with the corresponding sequencer specifications will not affect the detection results of this kit. The PCR reaction system composition (per well): *: 2 µL 5x KAPA HiFi buffer*; 0.3 µL 10 mM dNTPs; 0.5 µL DMSO; 0.2 µL KAPA HiFi Polymerase; 1 µL forward sequencing primer (5 µM); 1 µL reverse sequencing primer (5 µM); 3 µL nuclease-free water; 2 µL diluted first-round PCR product DNA. PCR reaction conditions: first 95°C-5min; then 98°C-20s, 50-60°C-30s, 72°C-1min, 10-20 cycles; finally 4°C hold.

### (3) Amplification product pooling and quality assessment

The second-round PCR amplification products were purified using AMPure XP beads. The purified DNA was quantified using the Qubit dsDNA High Sensitivity Assay Kit and qPCR method. After pooling amplification products from different samples in equimolar amounts, next-generation sequencing was performed on the corresponding sequencer.

### (4) Bioinformatics analysis of sequencing data

After sequencing data was generated, the fastqc software was used to check data quality, and the Trimmomatic software was used to remove sequencing adapters and low-quality bases and sequences, obtaining qualified post-QC sequences. Subsequently, the basmap methylation sequencing data alignment software was employed to align and analyze the qualified sequences against the target sequences of the amplification region. Then, the methylation level of CG bases on each target sequence was calculated for each sample, with the formula for methylation level being: methylation level at a C site = number of sequences supporting methylation/(number of sequences supporting methylation + number of sequences supporting non-methylation); based on the above formula, the methylation rate level at all methylation sites within the detection region for each sample could be obtained. For sites where the sequencing depth of the sequence was less than 50X , the methylation rate for that site in that sample was defined as a missing value NA and was no longer included in calculations.

### (5) Evaluation of targeted amplicon methylation sequencing performance

### ① Impact of DNA fragmentation degree on multiplex PCR amplification

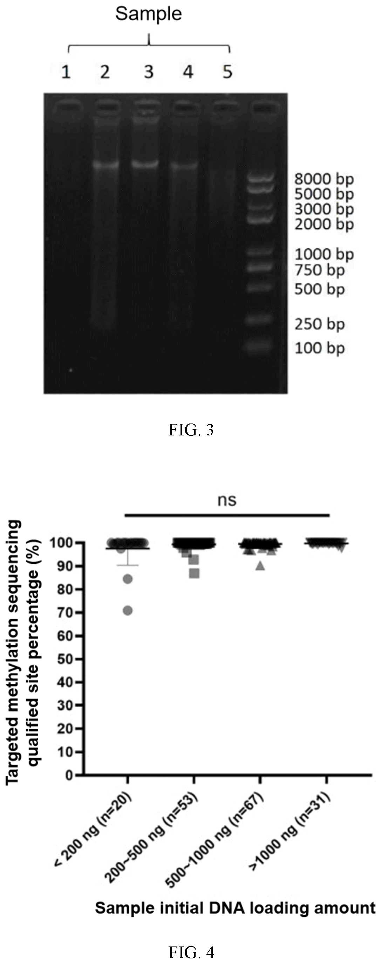

Since DNA from sources such as exfoliated cells often carries the risk of nucleic acid degradation and fragmentation, this disclosure tested five samples with varying degrees of fragmentation to observe the effect of DNA fragmentation on kit detection results. The results show that for severely fragmented samples, the percentage of qualified sites detected by the targeted methylation sequencing technology of this disclosure is above 99%, indicating that the detection method of this disclosure is largely unaffected by DNA fragmentation and can still yield reliable detection data under severe fragmentation conditions, as shown in FIG. 3 and Table 5.

**Table 5**

| Sample ID | DNA Loading Amount (ng) | Fragmentation Status | Percentage of Qualified Sites in Targeted Methylation Sequencing (%) |
|---|---|---|---|
| 1 | 8.57 | Yes | 99.48 |
| 2 | 118.67 | No | 99.90 |
| 3 | 89.67 | No | 99.90 |
| 4 | 90.73 | No | 100.00 |
| 5 | 38.80 | Yes | 99.53 |

### ②Effect of sample input amount on multiplex PCR amplification and sequencing

Samples non-invasively collected such as exfoliated cells often have large individual sampling heterogeneity and may also suffer from insufficient nucleic acid yield. Therefore, this disclosure tested the detection performance under different starting DNA input amounts. Results show that even when the DNA input is less than 200ng, the average percentage of qualified sites detected by targeted methylation sequencing can still reach 98%, with no statistical difference compared to the percentage of qualified sites in groups with higher starting DNA input amounts (FIG. 4). This indicates that the detection method of this disclosure can still obtain sufficient data even when only a small amount of nucleic acid is obtained from the test sample.

### ③Re-test consistency between multiplex targeted amplicon methylation sequencing and DNA methylation chip technology

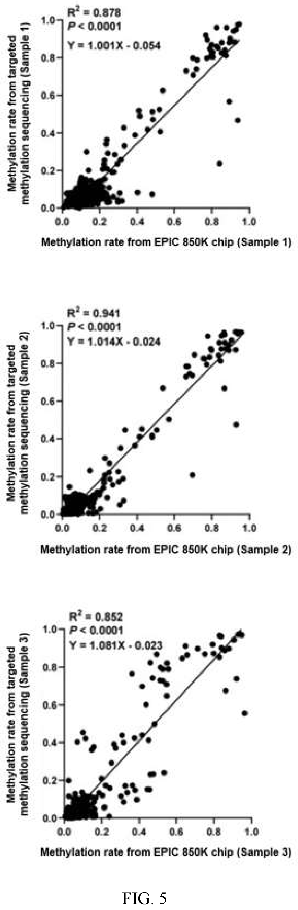

Different detection methods may exhibit certain deviations in the detection results of methylation sites. To evaluate how well the multiplex targeted amplicon methylation sequencing constructed in this application reflects the true methylation level in samples, three samples already tested by multiplex targeted amplicon methylation sequencing were also tested using the Illumina Epic 850K methylation chip. The Epic 850K methylation chip is currently one of the widely used and well-recognized DNA methylation detection methods. By comparing the methylation rates of methylation sites detected by both methods, the correlation of methylation rates between the two detection methods for these three re-tested samples was calculated. Results show good re-test consistency between the two methods for the three samples, with correlation coefficients R² of 0.878, 0.941, and 0.851 respectively, indicating a high positive correlation (FIG. 5). This demonstrates that the detection method of this disclosure can reflect the true methylation signals in the sample.

In summary, the multiplex targeted amplicon methylation sequencing detection method of this disclosure is compatible with common sample issues such as severe DNA fragmentation and low DNA input, can reflect the true methylation level in the sample, and has the feasibility to be developed into a detection kit. It is particularly advantageous when detecting samples with high individual heterogeneity, such as exfoliated cells and body fluid-derived samples.

### Example 3: Diagnostic performance of methylation markers for endometrial carcinoma in urine samples

Compared to blood samples and tissue samples, urine samples are completely non-invasive; compared to samples like pap smears and vaginal swabs, they are less affected by individual sampling techniques, do not require assistance from medical personnel, and have high public acceptance for this sampling method, enabling at-home collection. The detection samples in this example were urine from 107 endometrial carcinoma patients and 111 control participants collected at Sun Yat-sen University Cancer Center and the Affiliated Cancer Hospital of Guangzhou Medical University.

The detection method constructed in Example 2 of this application was used to extract DNA for the samples, DNA bisulfite modification was performed, a targeted methylation amplification library was constructed, and were sequenced on the Illumina platform. The sequencing data were processed through the aforementioned bioinformatics analysis pipeline to obtain the methylation levels of methylation sites within the amplification regions of 17 genes. After the data quality control process, information from 280 sites across the 17 genes was included in the analysis. Since the data underwent quality control filtering based on the sequencing depth of each gene, sites with sequencing depths below 50 reads were defined as missing. After this filtering, the final number of cases included per site ranged from 97 to 106, and the number of controls ranged from 105 to 111.

The genes included in the present disclosure were analyzed using targeted amplification methylation sequencing on independent samples. The methylation sites with the best diagnostic performance for endometrial carcinoma and their effect information are shown in Table 6. The number of methylation sites detected among the 17 genes ranged from 3 to 35. By constructing a Logistic model for the methylation sites within the same gene, the area under the curve (AUC) ranged from 0.788 to 0.886. Among them, three gene methylation markers, including GAD2, ADCYAP1, and C17orf64, had an AUC greater than 0.85; 16 markers, excluding CDO1, had an AUC greater than 0.8. Similar to the results in Example 1, CDO1 demonstrated excellent sensitivity at 91.3%; GAD2 and GRM7 had sensitivities above 80%; KCNA1 and ADCYAP1 had specificities above 90%. The AUCs of the best-performing methylation sites within the detection regions of the aforementioned 17 genes ranged from 0.730 to 0.814.

**Table 6**

| Gene | A | B | AUC¹ | Sensitivity (%) | Specificity (%) | C | AUC² |
|---|---|---|---|---|---|---|---|
| GAD2 | 102 | 109 | 0.886 | 87.3 | 78.0 | 33 | 0.768 |
| ADCYAP1 | 105 | 110 | 0.867 | 64.8 | 92.7 | 19 | 0.802 |
| C17orf64 | 101 | 106 | 0.864 | 74.3 | 88.7 | 29 | 0.780 |
| KCNA1 | 105 | 109 | 0.847 | 62.9 | 95.4 | 35 | 0.781 |
| RYR2 | 106 | 109 | 0.835 | 73.6 | 78.9 | 10 | 0.809 |
| ADARB2 | 104 | 108 | 0.833 | 62.5 | 89.8 | 14 | 0.784 |
| ZSCAN12 | 99 | 107 | 0.827 | 77.8 | 72.9 | 26 | 0.773 |
| KCNQ5 | 97 | 106 | 0.825 | 69.1 | 77.4 | 16 | 0.774 |
| STX16 | 106 | 110 | 0.825 | 73.6 | 80.0 | 10 | 0.798 |
| GRM7 | 106 | 111 | 0.823 | 82.1 | 70.3 | 11 | 0.786 |
| SORCS3 | 104 | 109 | 0.822 | 68.3 | 84.4 | 17 | 0.794 |
| ADHFE1 | 105 | 109 | 0.822 | 73.3 | 80.7 | 7 | 0.797 |
| GYPC | 106 | 108 | 0.821 | 70.8 | 82.4 | 27 | 0.730 |
| TMEM101 | 102 | 105 | 0.820 | 76.2 | 73.1 | 19 | 0.814 |
| NETO1 | 103 | 107 | 0.809 | 72.8 | 78.5 | 3 | 0.787 |
| TCTEX1D1 | 102 | 105 | 0.802 | 66.7 | 86.7 | 10 | 0.772 |
| CDO1 | 104 | 105 | 0.788 | 91.3 | 49.5 | 7 | 0.791 |

| | | | | | | | |
|---|---|---|---|---|---|---|---|
| Note: A is the sample size included in the modeling for the case group; B is the sample size included in the modeling for the control group; AUC¹ is the area under the curve for modeling all sites of this gene; C is the number of methylation sites identified in the amplified region; AUC² is the area under the curve for the methylation site with the best predictive performance. | | | | | | | |

Among the 17 methylated gene markers in this disclosure, GAD2, ADCYAP1, and C17orf64 demonstrate the best overall performance. GAD2 and others have advantages in sensitivity, while KCNA1 and ADCYAP1 excel in specificity. Therefore, the diagnostic efficacy of combined markers was further evaluated for 107 cases of endometrial carcinoma and 111 controls. Table 7 shows the discrimination performance when two-gene, three-gene, and four-gene markers are combined. Specifically, for two methylated gene marker panels, the panel of GAD2 and GYPC achieves the highest AUC of 0.928, with a sensitivity of 82.2% and specificity of 86.8%, showing a significant improvement over single methylated gene markers. Other pairwise gene panels may reach an AUC of 0.9, demonstrating excellent joint diagnostic performance. For three methylated gene marker panels, the panel of C17orf64, GAD2, and GYPC yields the highest AUC of 0.948, with a sensitivity of 85.4% and specificity of 91.2%, indicating a clear improvement in model sensitivity and accuracy compared to the two-marker model. Among them, panels involving GAD2, GYPC with C17orf64, ADCYAP1, TMEM101, and ZSCAN12 can achieve AUCs above 0.94. When ADARB2, CDO1, GRM7, KCNA1, NETO1, SORCS3, STX16, TCTEX1D1 genes are combined with C17orf64, GAD2, or GYPC, the AUC can also reach 0.93. Diagnostic performance can be further improved with four methylated gene marker panels, where the marker panel of ADCYAP1, GAD2, GYPC, and TMEM101 achieves an AUC of 0.959, with a sensitivity of 93.9%, and a specificity of 86.1%. In the better-performing four-marker panel models, GAD2, GYPC, and TMEM1 appear most frequently and are key genes in the panel models. Panels among genes such as GAD2, GYPC, ADCYAP1, TMEM101, C17orf64, ADHFE1, ZSCAN12, TCTEX1D1, STX16, RYR2, KCNQ5, SORCS3, RYR2, and KCNA1 achieve AUCs close to 0.95, demonstrating complementarity between genes.

**Table 7**

| Gene 1 | Gene 2 | Gene 3 | Gene 4 | AUC | Sensitivity (%) | Specificity (%) |
|---|---|---|---|---|---|---|
| C17orf64 | GAD2 | - | - | 0.928 | 83.3 | 88.5 |
| GAD2 | GYPC | - | - | 0.928 | 82.2 | 86.8 |
| ADCYAP1 | GAD2 | - | - | 0.923 | 82.0 | 88.9 |
| ADHFE1 | GAD2 | - | - | 0.917 | 79.0 | 88.8 |
| GAD2 | TMEM101 | - | - | 0.913 | 86.0 | 83.0 |
| ADARB2 | GAD2 | - | - | 0.911 | 89.2 | 82.4 |
| GAD2 | ZSCAN12 | - | - | 0.911 | 78.9 | 88.6 |
| GAD2 | RYR2 | - | - | 0.911 | 88.2 | 83.5 |
| GAD2 | STX16 | - | - | 0.910 | 81.2 | 86.1 |
| GAD2 | KCNQ5 | - | - | 0.909 | 79.6 | 91.3 |
| GAD2 | SORCS3 | - | - | 0.908 | 77.5 | 89.9 |
| GAD2 | NETO1 | - | - | 0.905 | 84.7 | 84.8 |
| GAD2 | GRM7 | - | - | 0.905 | 84.2 | 81.7 |
| ADCYAP1 | KCNA1 | - | - | 0.901 | 79.6 | 87.0 |
| GAD2 | TCTEX1D1 | - | - | 0.900 | 85.1 | 83.8 |
| CD01 | GAD2 | - | - | 0.898 | 86.0 | 85.4 |
| C17orf64 | GAD2 | GYPC | - | 0.948 | 85.4 | 91.2 |
| ADCYAP1 | GAD2 | GYPC | - | 0.947 | 88.0 | 86.7 |
| GAD2 | GYPC | TMEM101 | - | 0.943 | 87.0 | 87.5 |
| GAD2 | GYPC | ZSCAN12 | - | 0.941 | 90.4 | 83.3 |
| ADCYAP1 | C17orf64 | GAD2 | - | 0.940 | 80.2 | 92.3 |
| ADHFE1 | GAD2 | GYPC | - | 0.939 | 87.9 | 86.5 |
| C17orf64 | GAD2 | ZSCAN12 | - | 0.938 | 93.3 | 83.0 |
| ADCYAP1 | ADHFE1 | GAD2 | - | 0.937 | 87.8 | 86.8 |
| GAD2 | GYPC | RYR2 | - | 0.936 | 83.2 | 92.5 |
| ADCYAP1 | GAD2 | KCNQ5 | - | 0.936 | 87.9 | 82.5 |
| C17orf64 | GAD2 | STX16 | - | 0.935 | 80.2 | 90.3 |
| ADARB2 | GAD2 | GYPC | - | 0.935 | 84.2 | 89.5 |
| GAD2 | GYPC | KCNA1 | - | 0.934 | 84.2 | 89.6 |
| GAD2 | GYPC | TCTEX1D1 | - | 0.933 | 83.0 | 92.2 |
| C17orf64 | GAD2 | NETO1 | - | 0.933 | 85.4 | 88.5 |
| C17orf64 | GAD2 | SORCS3 | - | 0.932 | 86.5 | 87.5 |
| C17orf64 | CDO1 | GAD2 | - | 0.932 | 85.3 | 84.8 |
| C17orf64 | GAD2 | GRM7 | - | 0.932 | 83.3 | 87.5 |
| ADCYAP1 | GAD2 | GYPC | TMEM101 | 0.956 | 95.0 | 82.7 |
| C17orf64 | GAD2 | GYPC | ZSCAN12 | 0.954 | 87.6 | 92.9 |
| ADCYAP1 | GAD2 | GYPC | TCTEX1D1 | 0.953 | 89.9 | 85.1 |
| ADCYAP1 | ADHFE1 | GAD2 | GYPC | 0.952 | 85.7 | 91.3 |
| ADCYAP1 | GAD2 | GYPC | STX16 | 0.952 | 88.0 | 86.7 |
| ADCYAP1 | GAD2 | GYPC | RYR2 | 0.950 | 90.0 | 85.7 |
| ADCYAP1 | GAD2 | GYPC | KCNQ5 | 0.950 | 91.2 | 83.0 |
| C17orf64 | GAD2 | GYPC | SORCS3 | 0.950 | 86.5 | 92.2 |
| C17orf64 | GAD2 | GYPC | RYR2 | 0.949 | 88.5 | 89.2 |
| ADCYAP1 | GAD2 | GYPC | KCNA1 | 0.949 | 94.0 | 81.9 |
| ADCYAP1 | GAD2 | GYPC | NETO1 | 0.949 | 91.8 | 84.5 |
| C17orf64 | GAD2 | GRM7 | GYPC | 0.949 | 83.3 | 92.2 |
| ADARB2 | C17orf64 | GAD2 | GYPC | 0.947 | 83.3 | 94.1 |
| ADCYAP1 | CDO1 | GAD2 | GYPC | 0.947 | 87.9 | 86.3 |

When five methylated gene markers were included, all panels had AUCs above 0.857, with the optimal panel being GAD2, GYPC, ADCYAP1, TMEM101, and STX16, achieving an AUC of 0.960; the panel of GAD2, GYPC, ADCYAP1, C17orf64, and ZSCAN12 also had an AUC of 0.960. A total of 130 panels had AUCs above 0.95, and among these 130 panels, all 17 gene markers were repeatedly included in the models, fully demonstrating the importance of each gene in high-precision models.

For six-gene panel markers, all panels had AUCs above 0.872, with the optimal panel being ADCYAP1, C17orf64, GAD2, GYPC, TCTEX1D1, and ZSCAN12, achieving an AUC of 0.962; among these, the top 100 performing panels all had AUCs above 0.956, and all 17 gene markers were repeatedly included in the models, fully demonstrating the importance of each gene in high-precision models.

For seven-gene panel markers, all panels had AUCs above 0.884, with the optimal panel being ADCYAP1, C17orf64, GAD2, GYPC, TCTEX1D1, TMEM101, and ZSCAN12, achieving an AUC of 0.963; the panel ADARB2, ADCYAP1, GAD2, GYPC, STX16, TCTEX1D1, and TMEM101 also achieved the same diagnostic performance. Among the seven-gene panels, the top 100 performing panels all had AUCs above 0.960, and all 17 gene markers were repeatedly included in the models, fully demonstrating the importance of each gene in high-precision models.

For eight-gene panel markers, all panels had AUCs above 0.888, with the optimal panel being ADCYAP1, C17orf64, GAD2, GYPC, RYR2, TCTEX1D1, TMEM101, and ZSCAN12, achieving an AUC of 0.964; among the eight-gene panels, the top 100 performing panels all had AUCs above 0.961, and all 17 gene markers were repeatedly included in the models, fully demonstrating the importance of each gene in high-precision models.

In summary, the methylated gene markers or a panel thereof in this disclosure can effectively distinguish endometrial carcinoma from control individuals and can be used to prepare a kit for the diagnosis of endometrial carcinoma. Moreover, diagnosing endometrial carcinoma or precancerous lesions using panels of two or more of the above gene methylations can significantly improve sensitivity and specificity.

Notably, among the 100 endometrial carcinoma cases with clear pathological staging in the case group, stage I (very early-stage) patients accounted for as much as 73%, stage II patients accounted for 19%, and stage III and IV (mid-to-late stage) patients accounted for only 8%, demonstrating the excellent performance of the above markers for early-stage endometrial carcinoma, which can be used for early diagnosis of endometrial carcinoma.

### Example 4: Diagnostic performance of methylation markers for endometrial carcinoma in pap smear samples

To evaluate the diagnostic value of the 17 markers included in Example 1 for endometrial carcinoma in pap smear samples, the detection samples in this example were pap smears collected from 17 endometrial carcinoma patients and 14 control participants at Sun Yat-sen University Cancer Center and Foshan First People's Hospital. Pap smears are a relatively non-invasive sampling method that allows for self-sampling, offering advantages as a non-invasive diagnostic sampling option. The samples were processed using the detection method constructed in Example 2 of this application to extract DNA, perform DNA bisulfite modification, construct a targeted methylation amplification library, and sequence via the Illumina platform. Sequencing data were analyzed through the aforementioned bioinformatics pipeline to obtain methylation levels at methylation sites within the amplification regions of the 17 genes. After data quality control, information from 280 sites across the 17 genes was included in the analysis. Due to quality control filtering based on sequencing depth per gene, sites with a sequencing depth of less than 50 reads were defined as missing; after this filtering, the final number of cases analyzed per site ranged from 14 to 17, and controls ranged from 13 to 14.

The diagnostic performance of the genes included in this application for endometrial carcinoma in pap smear samples is shown in Table 8. The number of methylation sites detected among the 17 genes ranged from 3 to 35. By modeling methylation sites within the same gene using a Logistic model and selecting the optimal cutoff value, the accuracy of each gene in distinguishing individuals in the case and control groups was evaluated. Eleven gene methylation markers achieved perfect discrimination between endometrial carcinoma and control individuals, including ADARB2, ADCYAP1, C17orf64, GAD2, GYPC, KCNA1, KCNQ5, SORCS3, STX16, TMEM101, and ZSCAN12. Additionally, GRM7, TCTEX1D1, CDO1, and NETO1 correctly identified all controls, while RYR2 correctly identified all cases, with these gene methylation markers achieving 100% sensitivity, demonstrating strong advantages in recognizing controls. These results indicate that when using pap smear as the detection sample, the 17 gene methylation markers in this application exhibit excellent diagnostic capability for endometrial carcinoma.

**Table 8**

| Gene | A | B | C | D | AUC¹ | Sensitivity (%) | Specificity (%) | E | AUC² |
|---|---|---|---|---|---|---|---|---|---|
| ADARB2 | 17 | 14 | 17/17 | 14/14 | 1.000 | 100.0 | 100.0 | 14 | 0.893 |
| ADCYAP1 | 16 | 14 | 16/16 | 14/14 | 1.000 | 100.0 | 100.0 | 19 | 0.746 |
| C17orf64 | 16 | 14 | 16/16 | 14/14 | 1.000 | 100.0 | 100.0 | 29 | 0.886 |
| GAD2 | 17 | 14 | 17/17 | 14/14 | 1.000 | 100.0 | 100.0 | 33 | 0.863 |
| GYPC | 17 | 14 | 17/17 | 14/14 | 1.000 | 100.0 | 100.0 | 27 | 0.756 |
| KCNA1 | 17 | 14 | 17/17 | 14/14 | 1.000 | 100.0 | 100.0 | 35 | 0.895 |
| KCNQ5 | 14 | 11 | 17/17 | 11/11 | 1.000 | 100.0 | 100.0 | 16 | 0.890 |
| SORCS3 | 17 | 14 | 17/17 | 14/14 | 1.000 | 100.0 | 100.0 | 17 | 0.763 |
| STX16 | 17 | 14 | 17/17 | 14/14 | 1.000 | 100.0 | 100.0 | 10 | 0.893 |
| TMEM101 | 16 | 14 | 17/17 | 14/14 | 1.000 | 100.0 | 100.0 | 19 | 0.853 |
| ZSCAN12 | 15 | 13 | 17/17 | 13/13 | 1.000 | 100.0 | 100.0 | 26 | 0.795 |
| GRM7 | 17 | 14 | 16/17 | 14/14 | 0.962 | 94.1 | 100.0 | 11 | 0.805 |
| TCTEX1D1 | 17 | 14 | 15/17 | 14/14 | 0.962 | 88.2 | 100.0 | 10 | 0.796 |
| RYR2 | 17 | 14 | 17/17 | 12/14 | 0.950 | 100.0 | 85.7 | 10 | 0.794 |
| ADHFE1 | 17 | 14 | 12/17 | 13/14 | 0.853 | 70.6 | 92.9 | 7 | 0.784 |
| CD01 | 17 | 14 | 11/17 | 14/14 | 0.840 | 64.7 | 100.0 | 7 | 0.790 |
| NETO1 | 16 | 14 | 10/16 | 14/14 | 0.768 | 62.5 | 100.0 | 3 | 0.703 |

| | | | | | | | | | |
|---|---|---|---|---|---|---|---|---|---|
| Note: Item A is the sample size of the case group included for modeling; Item B is the sample size of the control group included for modeling; Item C is the correct discrimination of the case group, sample/total sample; Item D is the correct discrimination of the control group, sample/total sample; Item E is the number of methylation sites identified in the amplification region; AUC¹ is the area under the curve for modeling all sites of this gene; AUC² is the area under the curve for the methylation site with the best predictive performance. | | | | | | | | | |

### Example 5: Diagnostic performance of methylation markers for endometrial carcinoma in vaginal swab samples

To evaluate the diagnostic value of the 17 markers included in Example 1 for endometrial carcinoma in smear samples, the detection samples in this example were vaginal swabs collected from 17 endometrial carcinoma patients and 14 control participants at Sun Yat-sen University Cancer Center and Foshan First People's Hospital. Compared to smears, vaginal swab sampling has a shallower insertion depth and requires less assistance from medical personnel, making it a relatively non-invasive and simple sampling method. DNA was extracted from the samples using the detection method constructed in Example 2 of this application, followed by DNA bisulfite modification, construction of a targeted methylation amplification library, and sequencing on the Illumina platform. Sequencing data were processed through the aforementioned bioinformatics analysis pipeline to obtain methylation levels at sites within the amplification regions of the 17 genes. After the data quality control process, information from 280 sites across the 17 genes was included in the analysis. Since data were filtered for quality control based on the sequencing depth of each gene, sites with a sequencing depth of less than 50 reads were defined as missing. After this filtering, the final number of cases included for analysis per site ranged from 15 to 17, and the number of controls ranged from 11 to 14.

The diagnostic efficacy of the genes included in this application for endometrial carcinoma in pap smear samples is shown in Table 9. Among the 17 genes, the number of detected methylation sites ranged from 3 to 35. By modeling the methylation sites within the same gene using a Logistic model, the area under the curve (AUC) ranged from 0.874 to 1. Among these, 13 gene methylation markers achieved completely accurate discrimination between endometrial carcinoma and control individuals, with an AUC of 1, and both sensitivity and specificity of 100%. These gene methylation markers include ADARB2, ADCYAP1, C17orf64, GAD2, GYPC, KCNA1, KCNQ5, RYR2, SORCS3, STX16, TCTEX1D1, TMEM101, and ZSCAN12. Additionally, the AUCs for GRM7 and CDO1 genes were greater than 0.95, demonstrating excellent diagnostic performance. The AUCs for NETO1 and ADHFE1 were 0.929 and 0.874, respectively; these two gene methylation markers achieved a specificity of up to 100%, indicating strong capability in identifying controls. The above results indicate that when using vaginal swab samples as the detection sample, the 17 gene methylation markers in this application exhibit outstanding diagnostic capability for endometrial carcinoma.

**Table 9**

| Gene | A | B | C | D | AUC¹ | Sensitivity (%) | Specificity (%) | E | AUC² |
|---|---|---|---|---|---|---|---|---|---|
| ADARB2 | 17 | 14 | 17/17 | 14/14 | 1.000 | 100.0 | 100.0 | 14 | 0.897 |
| ADCYAP1 | 17 | 14 | 17/17 | 14/14 | 1.000 | 100.0 | 100.0 | 19 | 0.893 |
| C17orf64 | 17 | 14 | 17/17 | 14/14 | 1.000 | 100.0 | 100.0 | 29 | 0.931 |
| GAD2 | 17 | 14 | 17/17 | 14/14 | 1.000 | 100.0 | 100.0 | 33 | 0.952 |
| GYPC | 17 | 14 | 17/17 | 14/14 | 1.000 | 100.0 | 100.0 | 27 | 0.899 |
| KCNA1 | 17 | 14 | 17/17 | 14/14 | 1.000 | 100.0 | 100.0 | 35 | 0.943 |
| KCNQ5 | 15 | 12 | 15/15 | 12/12 | 1.000 | 100.0 | 100.0 | 16 | 0.914 |
| RYR2 | 17 | 14 | 17/17 | 14/14 | 1.000 | 100.0 | 100.0 | 10 | 0.916 |
| SORCS3 | 17 | 14 | 17/17 | 14/14 | 1.000 | 100.0 | 100.0 | 17 | 0.868 |
| STX16 | 17 | 14 | 17/17 | 14/14 | 1.000 | 100.0 | 100.0 | 10 | 0.929 |
| TCTEX1D1 | 17 | 14 | 17/17 | 14/14 | 1.000 | 100.0 | 100.0 | 10 | 0.954 |
| TMEM101 | 17 | 14 | 17/17 | 14/14 | 1.000 | 100.0 | 100.0 | 19 | 0.874 |
| ZSCAN12 | 15 | 11 | 17/17 | 14/14 | 1.000 | 100.0 | 100.0 | 26 | 0.864 |
| GRM7 | 17 | 14 | 16/17 | 13/14 | 0.979 | 94.1 | 92.9 | 11 | 0.857 |
| CDO1 | 17 | 14 | 15/17 | 14/14 | 0.975 | 88.2 | 100.0 | 7 | 0.922 |
| NETO1 | 17 | 14 | 12/17 | 14/14 | 0.929 | 70.6 | 100.0 | 3 | 0.903 |
| ADHFE1 | 17 | 14 | 11/17 | 14/14 | 0.874 | 64.7 | 100.0 | 7 | 0.800 |

| | | | | | | | | | |
|---|---|---|---|---|---|---|---|---|---|
| Note: Item A is the sample size of the case group included in the modeling; Item B is the sample size of the control group included in the modeling; Item C is correct discrimination of the case group, samples/total samples; Item D is correct discrimination of the control group, samples/total samples; Item E is the number of methylation sites identified in the amplified region; AUC¹ is the area under the curve for modeling all sites of this gene; AUC² is the area under the curve for the methylation site with the best predictive performance. | | | | | | | | | |

### Example 6: Detection performance of methylation markers in endometrial carcinoma tissues and adjacent samples

To evaluate the signal of the 17 markers included in Example 1 in endometrial carcinoma tissues, the detection samples in this example were 88 endometrial carcinoma tissues from patients with endometrial carcinoma, 34 adjacent tissues from patients with endometrial carcinoma, and 20 normal endometrial tissues from patients with benign fibroids, collected at the Sun Yat-sen University Cancer Center and the First People's Hospital of Foshan. The methylation signal in endometrial carcinoma tissues can reflect the true situation at the primary lesion, providing reliable validation for the marker signals in this application. The samples were processed using the detection method constructed in Example 2 of this application to extract DNA, perform DNA bisulfite modification, construct a targeted methylation amplification library, and sequence via the Illumina platform. Sequencing data were analyzed through the aforementioned bioinformatics pipeline to obtain the methylation levels of methylation sites within the amplified regions of the 17 genes. After data quality control, information from 280 sites across the 17 genes was included in the analysis.

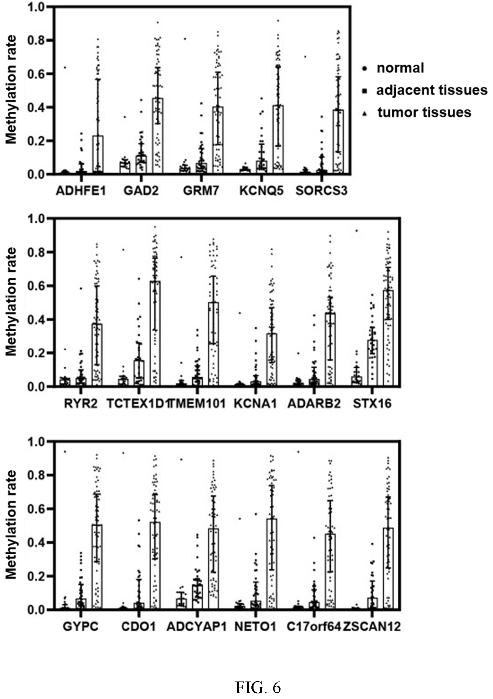

This disclosure comprehensively compared the methylation rate differences of the 17 gene methylation markers in this application between carcinoma and adjacent tissues, as well as between carcinoma and normal tissues, and used the signed-rank test for inter-group difference analysis. The results showed that, among the 280 methylation sites covered by the 17 genes, the methylation rates in carcinoma tissues were significantly higher than in normal tissues for all sites (P-value range: 6.4×10⁻¹¹ - 6.8×10⁻³, median P-value: 5.4×10⁻⁹); among these, 246 sites had significantly higher methylation rates in carcinoma than in adjacent tissues, accounting for 84%. FIG. 6 displays the methylation rate distribution of representative methylation sites from the 17 genes in normal, adjacent, and carcinoma tissues. As shown, the methylation rates at each methylation site are markedly higher in carcinoma tissues than in normal and adjacent tissues, particularly evident in genes such as GAD2, GRM7, SORCS3, TMEM101, KCNA1, GYPC, CDO1, and C17orf64, where the *P* value for the difference between carcinoma and adjacent tissues is less than 1×10⁻⁹.

### Example 7: Comparison of the Trend Changes in Methylation Markers Before and After Surgery

If the markers in this application can accurately reflect the presence of an endometrial carcinoma lesion, then the methylation signal should theoretically decrease significantly or even disappear after surgical removal of the lesion. To further add veracity evidence for the markers at this level, this example collected urine samples from 36 patients preoperatively and 3-5 days postoperatively at Sun Yat-sen University Cancer Center and Foshan First People's Hospital for dynamic monitoring of the markers in this application. Since endometrial carcinoma surgery typically involves total hysterectomy with bilateral salpingo-oophorectomy, collecting pap smears and vaginal swabs from postoperative patients would be inappropriate; therefore, less invasive urine samples were used as the monitoring sample type. As the tumor-associated methylation signals released by the lesion will cease after tumor resection, the methylation signal in postoperative patients should theoretically decrease.

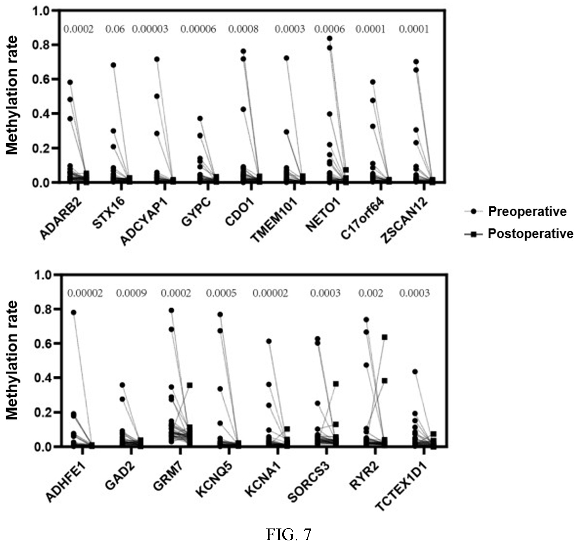

A comprehensive comparison was made of the methylation levels of the 17 gene methylation markers in this application across paired pre- and post-operative samples from the same patients, and a signed-rank paired test was used for intergroup difference analysis. The results revealed that among the 280 methylation sites covered by the detection of the 17 gene methylation markers, 235 sites showed significantly higher methylation rates in tumor tissues compared to adjacent tissues, accounting for 80%. FIG. 7 displays the distribution of methylation rates for representative methylation sites of the 17 genes in pre- and post-operative samples. As shown, compared to pre-operative samples, the methylation rates at each site in post-operative samples significantly decreased; with the exception of STX16, representative sites of the remaining 15 genes all reached significant levels, with *P* values ranging between 1.7×10⁻³ and 1.7×10⁻⁵. Overall, the pre-/post-operative differences in methylation sites such as ADHFE1, TMEM101, KCNA1, ADCYAP1, and GYPC were particularly notable, with *P* values below the 1×10⁻⁴ level.

The above embodiments are preferred implementations of the present disclosure, but the implementations of the present disclosure are not limited to these embodiments. Any other changes, modifications, substitutions, panels, or simplifications made without departing from the spirit and principles of the present disclosure should be regarded as equivalent replacement methods and are all included within the protection scope of the present disclosure.

## Claims

1. A methylated gene marker for early detection of endometrial carcinoma, wherein the methylated gene marker is selected from any at least one of the following methylation regions in any at least one of the target genes ADARB2ADCYAP1ADHFE1C17orf64CDO1, GAD2, GRM7GYPCKCNA1KCNQ5NETO1RYR2SORCS3STX16TCTEX1D1TMEM101and ZSCAN12:
ADARB2 gene: chr10: 1779052-1779183chr10: 1779756-1779910;
ADCYAP1 gene: chr18: 907522-907672 chr18: 908206-908385;
ADHFE1 gene: chr8: 67344466-67344599;
C17orf64 gene: chr17: 58498643-58498859 chr17: 58498879-58499081;
CDO1 gene: chr5: 115152259-115152414;
GAD2 gene: chr10: 26504914-26505063; chr10: 26506264-26506424 chr10: 26506907-26507068;
GRM7 gene: chr3: 6904068-6904274;
GYPC gene: chr2: 127413900-127414152;
KCNA1 gene: chr12: 5018675-5018893 chr12: 5019386-5019578;
KCNQ5 gene: chr6: 73331034-73331188 chr6: 73331252-73331411;
NETO1 gene: chr18: 70535858-70535996;
RYR2 gene: chr1: 237205894-237206034;
SORCS3 gene: chr10: 106400776-106400914 chr10106401432-106401587;
STX16 gene: chr20: 57225138-57225270;
TCTEX1D1 gene: chr1: 67218010-67218198;
TMEM101 gene: chr17: 42092213-42092341; and
ZSCAN12 gene: chr6: 28367209-28367379 chr6: 28367431-28367676.

2. A detection primer for early detection of endometrial carcinoma, configured to detect a methylation status of the methylation region of the gene marker described in claim 1, wherein nucleotide sequences of the detection primer are as follows:
ADARB2 gene:
chr10: 1779052-1779183 corresponds to detection primers SEQ ID NO:1-2;
chr10: 1779756-1779910 corresponds to detection primers SEQ ID NO:3-4;
ADCYAP1 gene:
chr18: 907522-907672 corresponds to detection primers SEQ ID NO:5-6;
chr18: 908206-908385 corresponds to detection primers SEQ ID NO:7-8;
ADHFE1 gene:
chr8: 67344466-67344599 corresponds to detection primers SEQ ID NO:9-10;
C17orf64 gene:
chr17: 58498643-58498859 corresponds to detection primers SEQ ID NO:11-12;
chr17: 58498879-58499081 corresponds to detection primers SEQ ID NO:13-14;
CDO1 gene:
chr5: 115152259-115152414 corresponds to detection primers SEQ ID NO:15-16;
GAD2 gene:
chr10: 26504914-26505063 corresponds to detection primers SEQ ID NO:17-18;
chr10: 26506264-26506424 corresponds to detection primers SEQ ID NO:19-20;
chr10: 26506907-26507068 corresponds to detection primers SEQ ID NO:21-22;
GRM7 gene:
chr3: 6904068-6904274 corresponds to detection primers SEQ ID NO:23-24;
GYPC gene:
chr2: 127413900-127414152 corresponds to detection primers SEQ ID NO:25-26;
KCNA1 gene:
chr12: 5018675-5018893 corresponds to detection primers SEQ ID NO:27-28;
chr12: 5019386-5019578 corresponds to detection primers SEQ ID NO:29-30;
KCNQ5 gene:
chr6: 73331034-73331188 corresponds to detection primers SEQ ID NO:31-32;
chr6: 73331252-73331411 corresponds to detection primers SEQ ID NO:33-34;
NETO1 gene:
chr18: 70535858-70535996 corresponds to detection primers SEQ ID NO:35-36;
RYR2 gene:
chr1: 237205894-237206034 corresponds to detection primers SEQ ID NO:37-38;
SORCS3 gene:
chr10: 106400776-106400914 corresponds to detection primers SEQ ID NO:39-40;
chr10: 106401432-106401587 correspond to detection primers SEQ ID NO:41-42;
STX16 gene:
chr20: 57225138-57225270 corresponds to detection primers SEQ ID NO:43-44;
TCTEX1D1 gene:
chr1: 67218010-67218198 corresponds to detection primers SEQ ID NO:45-46;
TMEM101 gene:
chr17: 42092213-42092341 corresponds to detection primers SEQ ID NO:47-48;
ZSCAN12 gene:
chr6: 28367209-28367379 corresponds to detection primers SEQ ID NO:49-50;
chr6: 28367431-28367676 corresponds to detection primers SEQ ID NO:51-52.

3. Use of a reagent for detecting a methylation region of a marker gene in preparation of a detection kit or device, wherein the detection kit or device is used for detecting, screening, or diagnosing endometrial carcinoma; and the methylation region of the marker gene is the methylation region of the marker gene according to claim 1.

4. The use according to claim 3, wherein the reagent comprises at least one of an antibody, a probe, a primer, and a mass spectrometry detection reagent specifically for detecting the methylation region of the marker gene, wherein the primer comprises the detection primer according to claim 2.

5. The use according to claim 3 or 4, wherein a detection sample of the detection kit or device comprises at least one of a body fluid sample, an exfoliated cell collection sample, and a tumor tissue sample.

6. The use according to claim 5, wherein the body fluid sample comprises at least one of urine and blood; and the exfoliated cell collection sample comprises at least one of a swab, a smear, and lavage fluid.

7. A multiplex PCR detection kit for early screening and diagnosis of endometrial carcinoma, comprising a reagent for detecting the methylation region of the marker gene according to claim 1; and
the reagent comprises at least one of an antibody, a probe, a primer, and a mass spectrometry detection reagent specifically for detecting the methylation region of the marker gene, wherein the primer comprises the detection primer according to claim 2.

8. Use of the methylated gene marker according to claim 1 in preparation of a product for diagnosing endometrial carcinoma.

9. Use of the multiplex PCR detection kit according to claim 7 in preparation of a product for diagnosing endometrial carcinoma.

10. The use according to claim 8 or 9, wherein the product comprises any one of a kit, a formulation, and a chip.
